# EUROPEAN PATENT SPECIFICATION

(11) **EP 2 205 973 B1**
(45) Date of publication and mention of the grant of the patent: **06.03.2013**
(21) Application number: 08833536.9
(22) Date of filing: 26.09.2008
(51) Int. Cl.: G01N 33/536

(54) **HIGH SENSITIVITY NANOTECHNOLOGY-BASED MULTIPLEXED BIOASSAY METHOD AND DEVICE**
AUF HOCHEMPFINDLICHER NANOTECHNOLOGIE BASIERENDES MULTIPLEX-BIOASSAY-VERFAHREN UND VORRICHTUNG
PROCÉDÉ ET DISPOSITIF DE BIOANALYSE MULTIPLEXÉE À BASE D'UNE NANOTECHNOLOGIE DE GRANDE SENSIBILITÉ

(30) Priority: 26.09.2007 IT PO20070023
(43) Date of publication of application: 14.07.2010
(73) Proprietor: Hospitex Diagnostics S.r.l., 50019 Sesto Fiorentino (IT)
(72) Inventor: LIGURI, Gianfranco, I-50141 Firenze (IT); TRISOLINI, Francesco, I-52025 Montevarchi (AR) (IT)
(86) International application number: PCT/IB2008/002526
(87) International publication number: WO 2009/040652

(56) References cited:
- WO-A1-02/44725
- WO-A2-03/095973
- PARK S-J ET AL: "ARRAY-BASED ELECTRICAL DETECTION OF DNA WITH NANOPARTICLE PROBES" SCIENCE, AMERICAN ASSOCIATION FOR THE ADVANCEMENT OF SCIENCE, WASHINGTON, DC; US, vol. 295, 22 February 2002 (2002-02-22), pages 1503-1506, XP001160895 ISSN: 0036-8075
- STOEVA SAVKA I ET AL: "Multiplexed detection of protein cancer markers with biobarcoded nanoparticle probes" JOURNAL OF THE AMERICAN CHEMICAL SOCIETY, vol. 128, no. 26, July 2006 (2006-07), pages 8378-8379, XP002571425 ISSN: 0002-7863
- TANSIL ET AL: "Nanoparticles in biomolecular detection" 1 February 2006 (2006-02-01), NANO TODAY, ELSEVIER, AMSTERDAM, NL, PAGE(S) 28 - 37 , XP005386420 ISSN: 1748-0132 figure 6
- REYNOLDS III, R.A., MIRKIN, C.A., LETSINGER, R.: "A gold nanoparticle/latex microsphere-based colorimetric oligonucleotide detection method", PURE APPL CHEM, vol. 72, no. 1-2, 2000, pages 229-235,
- ZHAO XIAOJUN ET AL: "Ultrasensitive DNA detection using highly fluorescent bioconjugated nanoparticles.", JOURNAL OF THE AMERICAN CHEMICAL SOCIETY, vol. 125, no. 38, 24 September 2003 (2003-09-24), pages 11474-11475, ISSN: 0002-7863

## Description

### Technical Field

This invention refers to a method and a device for the high sensitivity analysis of multiple analytes in biological samples.

To go into detail, the invention refers to a method and a device for the detection of complementary pairs of molecules, such as nucleic acid-nucleic acid (e.g. DNA-DNA, DNA-RNA, RNA-RNA) or protein-protein (e.g. antigen-antibody) pairs.

### Prior Art

It is well-known that during the last twenty years, molecular biology techniques have generated growing interest in the fields of biology and medicine. In particular, there is a strong interest in developing new bioassay techniques for a wide variety of applications, such as gene identification, gene mapping and DNA sequencing in medicine and in other diagnostic fields.

Amongst the more well-known techniques, Polymerase Chain Reaction (PCR) and its related modifications has been widely used both in research and in clinical diagnostics to specifically amplify and analyze traces of nucleic acids whose initial and terminal nucleotide sequences are known. This technique relies on the ability of the DNA polymerase enzyme to synthesize a new DNA strand from denatured DNA at certain temperatures. Ligase Chain Reaction (LCR), which relies on the action ofa thermostable ligase, represents the most common variation of PCR. These techniques are very sensitive and reliable, but are also very expensive and time-consuming. Moreover, they require skilled technicians and are not very easy to automate. Quantitative PCR (Q-PCR), which is based on the amplification of target DNA, is another common modification of PCR, but is only able to generate semi-quantitative data (number of copies of a DNA sequence in a sample) and can be affected by the presence of inhibitors and low copy number templates.

ELISA (Enzyme-Linked Immunosorbent Assay) and its related modifications is another technique that is frequently used in diagnostics. It is a type of immunologic analysis that, in biochemistry, is used to detect the presence of an antigen that is characteristic of a particular pathogenic organism, or to measure the concentration of antibodies in blood plasma (as in AIDS tests, for example). This technique relies on specific antigen-antibody reactions that are made visible by various procedures. In the last few years, many other ELISA-related techniques have been developed. One of them is the "Two-hybrid capture" technique, developed by Digene Corp. for the detection of human papilloma virus (HPV), which is able to detect RNA:DNA hybrids using an amplified chemiluminescent signal. These ELISA-related techniques, though sensitive and reliable, do not permit 'multiplexing' (the simultaneous detection of different subtypes (e.g. HPV subtypes) in the same reactor). The accuracy of such techniques can also be affected by the fluorescent signal used in detection, which tends to be fairly insensitive, by the low stability of dyes and by the influence of the physicochemical environment on signal intensity. Detection is often expensive, too.

At the start of the 1990s, DNA microarrays were becoming more important for parallel detection of DNA molecules, particularly in the field of biomedical research. A DNA microarray is a collection of microscopic DNA probes arrayed on a solid surface, such as glass or plastic, that bind to chemically suitable, complementary targets that have been previously amplified and tagged with a fluorescent molecule, which permits detection of fluorescent signals generated upon hybridization. These techniques therefore permit multiplexing, but require prior target DNA amplification to ensure optimal sensitivity, as in the case of RT-PCR-amplified mRNA detection. As such, they are affected by the aforementioned drawbacks of PCR. Microarrays also require very expensive scanners and sophisticated detection systems, which consist of specific focalized lasers that reveal every single micro-spot. Moreover, they are not easily automated and are therefore not very useful in diagnostics. In addition to this, they are also affected by the aforementioned limitations of fluorescent detection.

In recent years, new detection techniques derived from the well-known "Latex Agglutination" assay have been developed, with the aim of overcoming the analytical drawbacks associated with fluorescence-based techniques and PCR. In this case, the surface of the latex particles is coated with antibody (or antigen). When a suspension containing the complementary antigen (or antibody) to be detected is added to the particle suspension, it causes visible agglutination that allows the specific antigen or antibody to be detected by a dimensional scale "shift". However, these methods do not permit multiplexing, they require larger particles (micrometres in size) and are often only applied to specific antigens or antibodies. Numerous patents from this field can be found (US6200820, US5589401, US7122384, US7169556, US5175112), but most of these refer to very complex and mainly non-quantitative techniques.

A conductivity-based DNA detection method utilizing oligonucleotide-functionalized Au nanoparticles is described by Park et al. in "Array-Based Electrical Detection of DNA with Nanoparticles Probes", Science, vol. 295, pages 1503-1506. W003/095973A2 discloses an analyte detection method based on the use of Raman labelled nanoparticles that can be activate to provide SERS effect.

Both the above referred methods, to be effective, necessitate a silver enhancer solution.

A DNA detection method based on latex microsphere and gold nanoparticles is described by Reynolds III et al. in "A gold nanoparticle/latex microsphere-based colorimetric oligonucleotide detection method", Pure Appl. Chem., Vol. 72, Nos. 1-2, pp. 229-235, 2000. Gold and latex probes are linked together by the target DNA strand, generating a white-to red color change in a size-selective membrane through which excess or nonhybridized gold particles are separated from latex indicator probes.

### Disclosure of the Invention

Thus, the aim of this invention is to deliver an analytical biotechnology technique that is able to carry out detection of complementary pairs of molecules, such as nucleic acid-nucleic acid (DNA-DNA, DNA-RNA, RNA-RNA) or protein-protein pairs, without the drawbacks of other techniques.

In particular, the object of the invention is to provide a method and a device for the simultaneous and quantitative detection of different subtypes (multiplexing) of a given biological target system (virus, genetic mutations, etc.).

The aforementioned aims are achieved using a method and a device that exploits the interaction of non-biological nanoparticles with biological structures (such as nucleic acids and proteins) to determine dimensional increases, thereby permitting the detection of a biological target (virus, genetic mutations, etc.).

In particular, the proposed method and device make use of a microarray architecture of nanoparticles linked to molecular probes or specific antibodies, which are adsorbed onto a solid surface and preferably arranged in a monolayer. The aim of the system is to specifically detect complementary pairs of molecules, such as protein-protein pairs (e.g. antigen-antibody complexes) and DNA-DNA or DNA-RNA pairs (e.g. probe-target complexes). In addition to the use of fluorescent signals to detect and quantify these pairs, focalized laser sources coupled to a system of photosensors can preferably be used. Various detection strategies can then be exploited, such as image analysis or light scattering.

The advantages and technical characteristics of the invention will become more apparent from the following detailed description of a non-limiting example embodiment of it.

### Brief Description of the Drawings

In the drawings:
- Fig. 1 schematically illustrates the methodological purpose of the invention;
- Fig. 2 schematically illustrates the application of the invention to the detection of several subtypes of a biological system;
- Fig. 3 shows the magnification of detail A from Fig. 2.

### Preferred Embodiment of the Invention

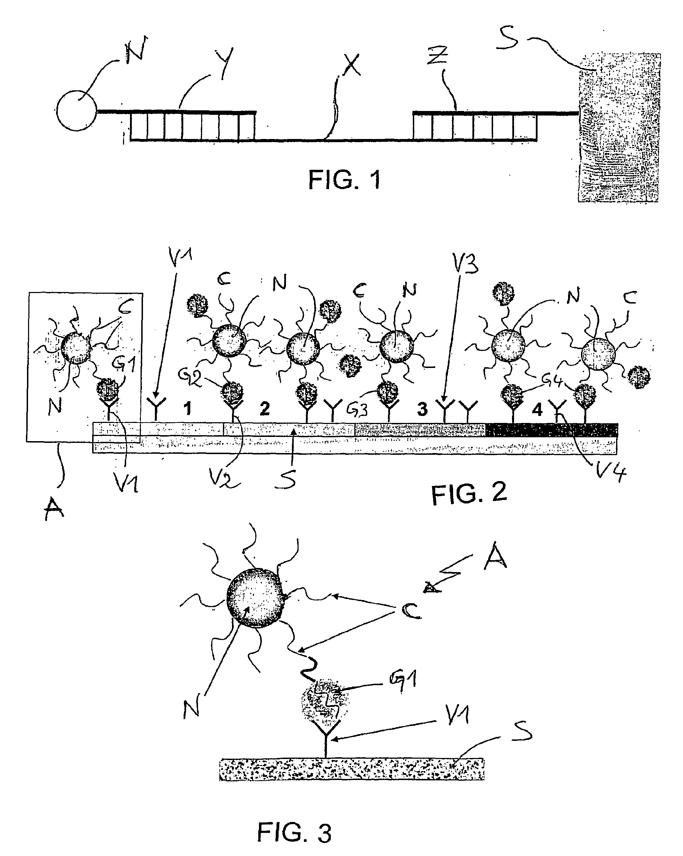

With reference to Fig. 1, to enable the detection of element X of a complementary pair of molecules (the target), binding to two other components Y,Z of the complementary element of the other pair is determined (in the illustrated example, two elements complementary to two different terminal portions of the target), one attached to a nanoparticle N and the other to a solid support S.

More specifically, in conformity with the invention, latex nanoparticles N, that are monodispersed in the suspension under test, specifically bind to the target X through complementary molecular probes Y that are attached to the nanoparticles. These nanoparticle-probe-target complexes diffuse by Brownian motion from the liquid suspension to the solid-liquid interface and dock onto the solid surface S (e.g. one of the internal surfaces of the tube containing the suspension) as a monolayer, resulting in the target X binding to other specific molecular probes Z (which are complementary to other portions of the target), that are also adsorbed onto the solid surface.

In this way, by docking different probes onto different areas of the solid surface (as shown below), an array of particles is created, with each area characterised by a particular probe-particle complex, thereby enabling the specific characterisation of several subtypes of a given biological system (multiplexing).

The proposed method guarantees rigidity and reliability, as well as rapid analysis and automation. Moreover, less time and money will be required to train personnel, compared to previous techniques.

Very sensitive analysis is obtained, not by a quantitative increase in material, as with PCR, but by exploiting the amplification signal that is generated when shifting from a molecular scale (nucleic acid or protein) to a microscopic one (latex nanoparticles): in fact, the "shift" in the dimensional scale is two- to three-fold in magnitude.

An additional process that increases the sensitivity of the test is the concentration effect that occurs when monodispersed particles diffuse though the liquid suspension and dock onto the solid surface as a monolayer.

Consequently, the method and the device according to the present invention enable two crucial aims in the medical diagnostic field to be fulfilled. The first of these is signal enhancement, in addition to better use of the solid space and selective concentration of the molecule to be analysed in a two-dimensional state, and secondly, the ability to carry out more simultaneous tests, in the same reactor and on the same sample (multiplexing), resulting in a considerable saving of time, money and biological material.

In addition to those mentioned previously, this last feature, in particular, is especially advantageous when it comes to detecting genetic mutations in human samples and can also be used to genotype viruses that are dangerous to humans.

In Figures 2 and 3, the detection of different genotypes (four in this case) of Human Papilloma Virus (HPV) is schematically illustrated using a method and a device that conforms to the proposed invention.

In this example, the genome (G1-G4) of the various HPV subtypes is the target to be detected. The probes (C) attached to the nanoparticles (N) are DNA probes that are complementary to the conserved regions of the HPV genome (and are therefore able to detect any HPV subtype), whereas the probes (V1-V4) attached to the solid support (S) are DNA probes that are complementary to the variable regions of the HPV genome (therefore, they can specifically detect a particular HPV subtype). As a consequence, this permits the spatial detection of various subtypes (multiplexing).

The system foresees the use of a plastic container (e.g. a tube), on one of whose internal surfaces (S) an area of approximately 1 cm² is selected and sub-divided into different cells (1-4). A different oligonucleotide (V1-V4), corresponding to a particular DNA probe that is complementary to the variable part of the genome of a specific HPV genotype, is attached to each cell.

A suspension containing latex nanoparticles (N) that are roughly 100-300 nm in size, which are linked to DNA probes (C) (previously attached to the nanoparticles) that are specific for the conserved portion of the HPV genome and, as a consequence, related to the various HPV subtypes, will then be added to the tube.

The biological sample is added to the tube so that qualitative and quantitative measurements of HPV can be made. In the presence of a specific target (e.g. G1) (i.e. the presence of a molecule of a certain HPV subtype), binding occurs between the target and a nanoparticle (N), through its conserved DNA portion (C), and with portion (I) of the internal surface (S) of the tube, through its variable DNA portion (V1), resulting in docking of the nanoparticles onto the internal surface of the tube (Fig. 3).

After washing (if necessary) to eliminate any unbound nanoparticles, qualitative detection of the various HPV subtypes present in the sample will be carried out (e.g. using light scattering techniques, image analysis or evanescent wave techniques) to determine the spatial distribution of nanoparticles that are bound to surface-attached type-specific oligonucleotide probes.

By producing calibration curves derived from experimental data, as well as by using a specific mathematical algorithm, it is also possible to perform quantitative detection of each HPV subtype present in the sample that was tested. The probabilistic and statistical correlation of the effective concentration of each specific subtype within the sample with the number of specific nanoparticles that are bound in a monolayer to the solid support avoids the possibility of under- or overestimating one or more subtypes.

## Claims

1. A high sensitivity multiplex bioassay method **characterised in that** it comprises the following steps:
- providing a plurality of latex or silica nanoparticles;
- applying at least one probe of a first type, that has affinity for at least one analyte, to each nanoparticle;
- applying a plurality of probes of at least one second type, that have affinity for said at least one analyte, to the surface of a support permitting the spatial detection of said analyte;
- mixing said plurality of nanoparticles with a fluid sample thereby determining the linkage of one ore more of said nanoparticles to said analyte, when contained in the sample, as a result of the binding occurring between the probe of the first type and the analyte;
- bringing said analyte-linked nanoparticles into contact with said support to bring about attachment to the surface as a result of the binding occurring between the probes of said second type and said analyte;
- detecting said analyte by analyzing the light signal generated by said surface-bound nanoparticles when illuminated.

2. A method according to claim 1, wherein said probes of the first type are complementary to a conserved region of said analyte and said probes of the second type are complementary to variable regions of said analyte.

3. A method according to claim 2, wherein said support permits the spatial detection of different subtypes of said analyte.

4. A method according to claim 3, wherein the surface of said support is divided into a plurality of areas and at least one probe, that is complementary to a different variable region of said analyte, is applied to each area, thereby resulting in the docking of nanoparticles linked to different subtypes of the analyte in different areas.

5. A method according to one of the preceding claims, wherein the light signal generated by the surface-bound nanoparticles is analyzed by means of light scattering techniques, image analysis or evanescent waves techniques.

6. A method according to one of the preceding claims, wherein said nanoparticles are 100 - 300 nm of size.

7. A high sensitivity multiplex bioassay device suitable for the method of claim 1 **characterised in that** it comprises:
- a receptacle in which a fluid sample can be analysed;
- a plurality of latex or silica nanoparticles dispersed in the fluid, with each nanoparticle attached to at least one probe of a first type having affinity for at least one analyte;
- a plurality of probes of at least a second type having affinity for said analyte and being attached to the surface of at least one wall of the receptacle;
- a focalized laser source for illuminating the nanoparticles that dock to the surface of the wall, resulting from the bindings occurring between the nanoparticle-bound probes and the analyte and between the analyte and the probes attached to the surface;
- photosensor means for detecting the light signal generated by said surface-bound nanoparticles when illuminated.

8. A device according to claim 7, wherein the light signal generated by the surface-bound nanoparticles is analyzed by means of light scattering techniques, image analysis or evanescent waves techniques.

9. A device according to claims 7 or 8, wherein the surface of said wall is divided into a plurality of areas, with each area being linked to at least one probe that is complementary to a different variable region of said analyte, thereby resulting in the docking of nanoparticles linked to different subtypes of the analyte in different areas.

10. A method and a device according to one of the preceding claims for detecting different genotypes of Human Papilloma Virus (HPV).

## Patentansprüche

1. Hochempfindliches Multiplex-Bioassay-Verfahren, **dadurch gekennzeichnet, dass** es die folgenden Schritte umfasst:
- Bereitstellen einer Vielzahl von Latex- oder Silica-Nanopartikeln;
- Aufbringen mindestens einer Sonde einer ersten Art, die Affinität für mindestens einen Analyten aufweist, auf jeden Nanopartikel;
- Aufbringen einer Vielzahl von Sonden von mindestens einer zweiten Art, die Affinität für den mindestens einen Analyten aufweisen, auf die Oberfläche eines Trägers wodurch die räumliche Erfassung des Analyten ermöglicht wird;
- Mischen der Vielzahl von Nanopartikeln mit einer Fluidprobe, wodurch die Bindung von einem oder mehreren der Nanopartikel an den Analyten bestimmt wird, wenn in der Probe enthalten, als Ergebnis der Bindung, die zwischen der Sonde der ersten Art und dem Analyten erfolgt;
- Inkontaktbringen der analytgebundenen Nanopartikel mit dem Träger, um Kopplung an die Oberfläche als Ergebnis der Bindung, die zwischen den Sonden der zweiten Art und dem Analyten erfolgt, zu bewirken;
- Erfassen des Analyten durch Analysieren des Lichtsignals, das durch die oberflächengebundenen Nanopartikel erzeugt wird, wenn sie beleuchtet werden.

2. Verfahren nach Anspruch 1, wobei die Sonden der ersten Art zu einer konservierten Region des Analyten komplementär sind und die Sonden der zweiten Art zu variablen Regionen des Analyten komplementär sind.

3. Verfahren nach Anspruch 2, wobei der Träger die räumliche Erfassung verschiedener Subtypen des Analyten ermöglicht.

4. Verfahren nach Anspruch 3, wobei die Oberfläche des Trägers in eine Vielzahl von Bereichen unterteilt ist und mindestens eine Sonde, die zu einer anderen variablen Region des Analyten komplementär ist, auf jeden Bereich aufgebracht wird, wodurch sich das Andocken von Nanopartikeln, die an verschiedene Subtypen des Analyten gebunden sind, in verschiedenen Bereichen ergibt.

5. Verfahren nach einem der vorhergehenden Ansprüche, wobei das Lichtsignal, das durch die oberflächengebundenen Nanopartikel erzeugt wird, mittels Lichtstreuungsmethoden, Bildanalyse oder evaneszenten Wellenmethoden analysiert wird.

6. Verfahren nach einem der vorhergehenden Ansprüche, wobei die Nanopartikel eine Größe von 100 - 300 nm aufweisen.

7. Hochempfindliche Multiplex-Bioassay-Vorrichtung, die für das Verfahren nach Anspruch 1 geeignet ist, **dadurch gekennzeichnet, dass** sie Folgendes aufweist:
- Ein Behältnis, in dem eine flüssige Probe analysiert werden kann;
- eine Vielzahl von Latex- oder Silica-Nanopartikeln, die in der Flüssigkeit aufgeschlämmt sind, wobei jedes Nanopartikel, das mit mindestens einer Sonde einer ersten Art gekoppelt ist, Affinität für mindestens einen Analyten aufweist;
- eine Vielzahl von Sonden mindestens einer zweiten Art, die Affinität für den Analyten aufweisen und die an die Oberfläche von mindestens einer Wand des Behälters gekoppelt sind;
- eine fokussierte Laserquelle zum Beleuchten der Nanopartikel, die an der Oberfläche der Wand, als Ergebnis der Bindungen, die zwischen den nanopartikelgebundenen Sonden und dem Analyten und zwischen dem Analyten und den Sonden, die an der Oberfläche gekoppelt sind, erfolgen, andocken;
- Fotosensormittel zum Erfassen des Lichtsignals, das durch die oberflächengebundenen Nanopartikel erzeugt wird, wenn sie beleuchtet werden.

8. Vorrichtung nach Anspruch 7, wobei das Lichtsignal, das durch die oberflächengebundenen Nanopartikel erzeugt wird, mittels Lichtstreuungsmethoden, Bildanalyse oder evaneszenten Wellenmethoden analysiert wird.

9. Vorrichtung nach den Ansprüchen 7 oder 8, wobei die Oberfläche der Wand in eine Vielzahl von Bereichen unterteilt ist wobei jeder Bereich mit mindestens einer Sonde verbunden ist, die zu einer anderen variablen Region des Analyten komplementär ist, wodurch sich das Andocken von Nanopartikeln, die an verschiedene Subtypen des Analyten gebunden sind, in verschiedenen Bereichen ergibt.

10. Verfahren und Vorrichtung nach einem der vorhergehenden Ansprüche zur Erfassung verschiedener Genotypen des menschlichen Papilloma-Virus (HPV).

## Revendications

1. Une méthode de titrage biologique multiple haute sensibilité, **caractérisée par le fait qu'**elle comprend les étapes suivantes :
- la fourniture d'une série de nanoparticules de latex ou de silice ;
- l'application d'au moins une sonde d'un premier type ayant une affinité avec, au moins, un analyte de chaque nanoparticule ;
- l'application d'une série de sondes, d'au moins d'un seconde type ayant une affinité avec, au moins, ledit analyte, sur la surface d'un support permettant la détection dudit analyte dans l'espace ;
- le mélange de ladite série de nanoparticules avec un échantillon liquide déterminant ainsi le jumelage d'une ou plusieurs desdites nanoparticules, si contenues dans l'échantillon, comme conséquence du couplage entre la sonde du premier type et l'analyte ;
- la mise en contact desdites nanoparticules liées à l'analyte avec ledit support afin de provoquer l'attachement à la surface, comme conséquence du couplage entre les sondes du second type et ledit analyte ;
- la détection dudit analyte par l'analyse du signal lumineux généré par les nanoparticules liées à la surface dès lors qu'elles sont illuminées.

2. Une méthode suivant la revendication 1 où lesdites sondes du premier type sont complémentaires à une zone conservée dudit analyte et lesdites sondes du second type sont complémentaires aux zones variables dudit analyte.

3. Une méthode suivant la revendication 2 où ledit support permet la détection dans l'espace des différents sous-types dudit analyte.

4. Une méthode suivant la revendication 3 où la surface dudit support est divisée en plusieurs zones et, au moins, une sonde qui est complémentaire à l'une des zones variables dudit analyte, est appliquée à chacune des zones, donnant ainsi comme résultat la fixation des nanoparticules liées aux différents sous-types d'analyte dans les différentes zones.

5. Une méthode suivant l'une des revendications précédentes où le signal lumineux généré par les nanoparticules liées à la surface est analysé par le biais de techniques de diffusion de la lumière, d'analyse d'image et d'ondes évanescentes.

6. Une méthode suivant l'une des revendications précédentes où lesdites nanoparticules ont une taille de 100 - 300 mm.

7. Un dispositif de titrage biologique multiplexe haute sensibilité adapté à la méthode citée dans la revendication 1, **caractérisé par le fait qu'**il comprend :
- un réceptacle dans lequel l'échantillon liquide peut être analysé :
- une série de nanoparticules de latex ou de silice dispersées dans le liquide, et dont chaque nanoparticule est attachée à, au moins, une sonde d'un premier type ayant une affinité avec, au moins, un analyte ;
- une série de sondes, au moins, du second type ayant une affinité avec ledit analyte et étant attachées à la surface d'au moins une paroi du réceptacle ;
- une source laser concentrée pour l'illumination des nanoparticules présentes sur la surface de la paroi, résultant des fixations ayant eu lieu entre les sondes liées aux nanoparticules et l'analyte et entre l'analyte et les sondes attachées à la surface ;
- des moyens photosenseurs pour la détection du signal lumineux généré par les nanoparticules liées à la surface dès lors qu'elles sont illuminées.

8. Un dispositif suivant la revendication 7 où le signal lumineux généré par les nanoparticules liées à la surface est analysé par le biais de techniques de diffusion de la lumière, d'analyse d'image et d'ondes évanescentes.

9. Un dispositif suivant la revendication 7 ou 8 où la surface de ladite paroi est divisée en plusieurs zones, avec, au moins, chaque zone liée à, au moins, une sonde qui est complémentaire à l'une des différentes zones variables dudit analyte, donnant ainsi la fixation des nanoparticules liées aux différents sous-types d'analyte dans les différentes zones.

10. Une méthode et un dispositif suivant l'une des revendications précédentes pour la détection des différents génotypes du virus du papillome humain (HPV).
